# EUROPÄISCHE PATENTSCHRIFT

(11) **EP 0 228 988 B1**
(45) Veröffentlichungstag und Bekanntmachung des Hinweises auf die Patenterteilung: **13.04.1994**
(21) Anmeldenummer: 86730209.3
(22) Anmeldetag: 15.12.1986
(51) Int. Cl.: A61K 37/47, A61K 37/54

(54) **Trombosebehandlung mit Fibrinolytika und Prostacyclinen**
Treatment of thrombosis with fibrinolytics and prostacyclins
Traitement de la thrombose par des fibrinolytiques et des prostacyclines

(30) Priorität: 13.12.1985 DE 3544663
(43) Veröffentlichungstag der Anmeldung: 15.07.1987
(73) Patentinhaber: SCHERING AKTIENGESELLSCHAFT, 13342 Berlin (DE)
(72) Erfinder: Baldus, Berthold, D-1000 Berlin 28 (DE); Maass, Bernhard, D-1000 Berlin 21 (DE); Müller, Bernd, Dr., D-1000 Berlin 15 (DE); Witt, Werner, D-1000 Berlin 30 (DE)

(56) Entgegenhaltungen:
- CHEMICAL ABSTRACTS, Band 103, Nr. 13, 30 September 1985, Columbus, OH (US); W.A. SCHUMACHER et al., Seite 36, Nr. 98516r#

## Beschreibung

Die Erfindung betrifft ein Kombinationserzeugnis zur Thrombosebehandlung, enthaltend ein Fibrinolytikum und ein Carbacyclin-Analogon.

Nach K.H. Gold et al. Circulation 68, I-50 bis I-54 (1983) und W. Ganz et al. Amer. Heart J. 101,4 (1981) stellt die Rethrombosierung nach zunächst erfolgreicher Thrombolyse weiterhin ein Problem bei der fibrinolytischen Therapie dar. Als Pathomechanismen diskutieren sie, daß an der ursprünglich thrombosierten Stelle nach Auflösung von Thromben eine geschädigte und damit thrombogene Gefäßinnenwand sowie in vielen Fällen eine durch Stenosen induzierte Blutungsanomalie zurückbleibt. Thrombogene Oberflächen und eine rheologische Störung führen aber zu erneuter Aktivierung von plasmatischem Garinnungssystem und Thrombozyten.

H.P. Klöcking beschreibt in seinem Artikel "Pharmacology of Streptokinase" (aus dem Handbuch "Experimental Pharmacology 46:Fibrinolytics and Antifibrinolytics, F.Markward (ed.), Springer-Verlag, Berlin-Heidelberg-New York, 1978, 151-177), daß speziell nach Thrombolyse mit Streptokinase und Urokinase, in geringerem Umfang aber auch nach t-PA und pro-Urokinase, Spaltprodukte von Fibrinogen und Fibrin auftreten, die zur erhönten Aggregationsbereitschaft von Thrombozyten führen und damit die rasche Neubildung von Plättchenthromben an der ursprünglichen Stelle eines lysierten Thrombus begünstigen.

Die bisher klinisch gebräuchliche Prophylaxe einer raschen Rethrombisierung bestand in der Hemmung der plasmatischen Gerinnung durch Heparin. E. Hiller äußert sich hierzu in der Münchner medizin. Wochenschrift 126 , 13 (1984) und hält eine Prophylaxe mit Heparin für problematisch, weil Plättchen-induzierte Rethrombosen dadurch nicht verhindert, sondern nach seinen Befunden sogar eher begünstigt werden. Nach Klöcking ist das plasmatische Gerinnungssystem besonders nach Thrombolyse mit Streptokinase oder Urokinase durch den Verbrauch an Gerinnungsfaktoren nicht mehr voll funktionsfähig. Durch die zusätzliche Gabe von Heparin wird die Gefahr von Blutungen begünstigt.

Welcher Anteil den Thrombozyten und welcher dem plasmatischen Gerinnungssystem bei der Rethrombosierung zukommt, wird zwar häufig diskutiert, konnte aber bisher nicht geklärt werden.

Schuhmacher et al. (J. of Cardiovascular Pharmacology, 7, 739-746 (1985) haben Heparin bzw. die Kombination aus Heparin + Prostacyclin (PGI₂) hinsichtlich einer möglichen Verstärkung der Streptokinase-induzierten Thrombolyse untersucht. Sie kommen zu dem Ergebnis, daß der Zusatz von Prostacyclin keine signifikante Verbesserung des erneuten Durchflusses bewirkt.

Es wurde nun gefunden, daß die Rethrombose nach Thrombolyse verhindert werden kann, wenn man Fibrinolytika und Carbacyclinanaloga gemeinsam für die Auflösung von Thromben verwendet.

Aktivierung, Aggregation und Adhäsion von Thrombozyten kommt bei der Rethrombosierung nach erfolgreicher Thrombolyse die entscheidende Rolle zu. Durch die Thrombozytenaggregationshemmende Wirkung der Carbacyclinanaloga tritt von vornherein keine Rethrombosierung mehr auf.

Als Carbacyclinanaloga kommen für die erfindungsgemäße Verwendung infrage:
Carbacyclinderivate, wie sie in den DE-OS 2845770, 3204443, 3226550 und 3306123 beschrieben werden.
Bevorzugte Carbacycline für die erfindungsgemäße Verwendung sind
5-{(E)-(1S,5S,6R,7R)-7-Hydroxy-6-[(E)-(3R,4RS)-3-hydroxy-4-methyl-oct-1-en-6-inyl]-bicyclo[3.3.0]octan-3-yliden}-pentansäure (Iloprost),
(5E)-(16RS)-13,14-Didehydro-16,20-dimethyl-18,18,19,19-tetradehydro-6a-carbaprostaglandin-I₂,
(5E)-(16RS)-13,14-Didehydro,1a,1b-dihomo-16,20-dimethyl-3-oxa-18,18,19,19-tetradehydro-6a-carbaprostaglandin-I₂ und
5-{(E)-(1S,5S,6R,7R)-7-Hydroxy-6-[(3S,4S)-3-hydroxy-4-methylnona-1,6-diinyl]-bicylo[3.3.0]octan-3-yliden}-3-oxapentansäure.

Die genannten Carbacycline werden in Mengen eingesetzt, die im Bereich der sonst für die Thrombozytenaggregationshemmung üblichen Mengen liegen. Die entsprechend vorliegender Erfindung anzuwendende Menge hängt vom Umfang der Thrombose und der zu erwartenden Rethrombosierung ab.
Als Fibrinolytika kommen Streptokinase, Urokinase und t-PA sowie pro-Urokinase und pro t-PA in Betracht.
Die erfindungsgemäße Kombination wird vorzugsweise infundiert oder intramuskulär injiziert. Bei parenteraler Anwendung von Streptokinase, Urokinase, t-PA pro-Urokinase und pro-tPA können die in der Kombination eingesetzten Carbacycline auch oral verabreicht werden.

Eine typische Dosis für die Anwendung am Menschen für den Carbacyclinteil dieser Erfindung ist, abhängig von der Applikationsart, 10 pg bis 1 mg (i.v.) oder 10 µg bis 500 mg (p.o.), bevorzugt 0,1 bis 100 µg, besonders bevorzugt 0,01 - 10 µg/kg Körpergewicht/Minute an Iloprost i.v. oder eine biologisch äquivalente Menge eines anderen Carbacyclin-Analogen. Eine typische Dosis des Fibrinolytikums ist 5.000 bis 10.000.000 IU an Streptokinase, bevorzugt 50.000 - 5.000.000, besonders bevorzugt 250.000 - 2.000.000 IU i.v. oder einer biologisch äquivalenten Menge eines anderen Fibrinolytikums. Das Gewichtsverhältnis von Fibrinolytikum zu Carbacyclinanalogen ist etwa 10⁸ : 1 bis 10² : 1, bevorzugt 10⁵ : 1 bis 10³ : 1.

### Beispiel

An 10 narkotisierten Beagle-Hunden (Narkose mit Tramadol, 2mg/kg i.v. + 0,5 mg/kg/h i.v.; Imbretil^{R}, 0,33 mg/kg i.v., N₂O/O₂ 80:20) wurden Katheter zur Infusion und elektrischen Stimulation über periphere Arterien in die linke deszendierende Koronararterie plaziert. Zur Thrombusinduktion wurde kontinuerlich mit einer Stromstärke von 250 µA intrakoronar stimuliert. In 15-Min.-Abständen wurde das Anwachsen eines Koronarthrombus bis zum vollständigen Verschluß durch intrakoronare Injektion des Kontrastmittels Urografin^{R} 76 bei Röntgenkontrolle bewertet. Bei vollständigem Verschluß der Koronararterie wurde bis zur röntgenologisch festgestellten Wiederdurchströmung Streptokinase (250 I.U./kg/min) intrakoronar infundiert. Bei 5 Tieren wurde nur Streptokinase, bei den anderen 5 Tieren zusätzlich das Prostacyclinanalogon Iloprost (50 ng/kg/min) intravenös infundiert.

### Ergebnisse

Bei 10/10 Tieren führte die Streptokinase-Infusion innerhalb von im Mittel 60 Min. zur Wiedereröffnung der zuvor vollständig thrombotisch verschlossenen Koronararterie.

Bei 5/5 Hunden, die ausschließlich Streptokinase erhielten, trat im Mittel 21 Min. nach Wiederöffnung spontan ein vollständiger Wiederverschluß durch Rethrombosierung auf.

Wurde zusätzlich zu Streptokinase Iloprost intravenös infundiert, so trat bei keinem von 5 Tieren innerhalb dieses Zeitraums eine Rethrombosierung auf.

Auch an einem Hund, der zusätzlich zu Streptokinase an Stelle von Iloprost 5-{(E)-(1S,5S,6R,7R)-7-Hydroxy-6-[(3S,4S)-3-hydroxy-4-methyl-nona-1,6-diinyl]-bicyclo[3.3.0]octan-3-yliden} -3-oxapentansäure in der Dosierung 5 ng/kg/min. intravenös erhielt, trat keine Rethrombosierung auf.

## Patentansprüche (Patentansprüche für folgende(n) Vertragsstaat(en): BE, CH, DE, FR, GB, IT, LI, LU, NL, SE)

1. Kombinationserzeugnis zur Thrombosebehandlung, enthaltend ein Fibrinolytikum und ein Carbacyclinanalogon.

2. Erzeugnis gemäß Anspruch 1, dadurch gekennzeichnet, daß Fibrinolytikum und Carbacyclinanalogon in getrennten Dosiseinheiten vorliegen.

3. Erzeugnis gemäß Anspruch 1, dadurch gekennzeichnet, daß Fibrinolytikum und Carbacyclinanalogon in einem Gewichtsverhältnis 10⁸:1 bis 10²:1 zueinander stehen.

4. Erzeugnis gemäß Anspruch 1, dadurch gekennzeichnet, daß es eine Carbacyclin-Dosiseinheit 10ng bis 1mg Iloprost oder eine biologisch äquivalente Menge eines anderen Carbacyclinsenthält.

5. Erzeugnis gemäß Anspruch 1, dadurch gekennzeichnet, daß es eine Fibrinolytikum-Dosiseinheit 5000 IE bis 10 Mill.I.E. Streptokinase oder eine biologisch äquivalente Menge eines anderen Fibrinolytikums enthält.

## Patentansprüche (Patentansprüche für folgende(n) Vertragsstaat(en): GR, AT, ES)

1. Verfahren zur Herstellung eines Kombinationserzeugnisses zur Thrombosebehandlung, dadurch gekennzeichnet, dass ein Fibrinolytikum und ein Carbocyclinanalogon in getrennten Dosiseinheiten vorbereitet und beide Komponenten so gemischt werden, dass das Fibrinolytikum und das Carbacyclinanalogon in einem Gewichtsverhältnis von 10⁸.1 bis 10²:1 zueinander stehen.

2. Verfahren gemäss Anspruch 1, dadurch gekennzeichnet, dass eine Carbacyclin-Dosiseinheit vorbereitet wird, welche zwischen 10ng und 1mg Iloprost oder eine biologisch äquivalente Menge eines anderen Carbacyclins enthält.

3. Verfahren gemäss Anspruch 1, dadurch gekennzeichnet, dass eine Fibrinolytikum-Dosiseinheit vorbereitet wird, die zwischen 5000 IE bis 10 Mill.I.E. Streptokinase oder eine biologisch äquivalente Menge eines anderen Fbrinolytikums enthält.

## Claims (Claims for the following Contracting State(s): BE, CH, DE, FR, GB, IT, LI, LU, NL, SE)

1. Combination product for the treatment of thrombosis, comprising a fibrinolytic agent and a carbacyclin analogue.

2. Product according to claim 1, characterised in that the fibrinolytic agent and the carbacyclin analogue are present in separate dosage units.

3. Product according to claim 1, characterised in that the fibrinolytic agent and the carbacyclin analogue are in a weight ratio with respect to one another of from 10⁸:1 to 10²:1.

4. Product according to claim 1, characterised in that it contains as carbacyclin dosage unit from 10 ng to 1 mg of Iloprost or a biologically equivalent amount of another carbacyclin.

5. Product according to claim 1, characterised in that it contains as fibrinolytic agent dosage unit from 5000 IU to 10 million IU of streptokinase or a biologically equivalent amount of another fibrinolytic agent.

## Claims (Claims for the following Contracting State(s): GR, AT, ES)

1. Process for the preparation of a combination product for the treatment of thrombosis, characterised in that a fibrinolytic agent and a carbacyclin analogue are prepared in separate dosage units and the two components are so mixed that the fibrinolytic agent and the carbacyclin analogue are in a weight ratio with respect to one another of from 10⁸:1 to 10²:1.

2. Process according to claim 1, characterised in that a carbacyclin dosage unit is prepared that contains from 10 ng to 1 mg of iloprost or a biologically equivalent amount of another carbacyclin.

3. Process according to claim 1, characterised in that a fibrinolytic agent dosage unit is prepared that contains from 5000 IU to 10 million IU of streptokinase or a biologically equivalent amount of another fibrinolytic agent.

## Revendications (Revendications pour l'(les) Etat(s) contractant(s) suivant(s): BE, CH, DE, FR, GB, IT, LI, LU, NL, SE)

1. Produit destiné au traitement de thromboses comprenant une association d'un fibrinolytique et d'un analogue de carbacyclines.

2. Produit selon la revendication 1, caractérisé en ce que le fibrinolytique et l'analogue de carbacycline sont en doses unitaires séparées.

3. Produit selon la revendication 1, caractérisé en ce que le fibrinolytique et l'analogue de carbacyclines sont dans un rapport pondéral entre eux compris entre 10⁸ et 10².

4. Produit selon la revendication 1, caractérisé en ce qu'il contient une dose unitaire de carbacycline de 10 ng à 1 mg d'iloprost ou une quantité biologiquement équivalente d'un autre analogue de carbacycline.

5. Produit selon la revendication 1, caractérisé en ce qu'il contient une dose unitaire de fibrinolytique de 5000 EI à 10Mill EI de streptokinase ou une quantité biologiquement équivalente d'un autre fibrinolytique.

## Revendications (Revendications pour l'(les) Etat(s) contractant(s) suivant(s): GR, AT, ES)

1. Procédé de préparation d'un produit associé pour le traitement de thromboses, procédé caractérisé en ce que l'on prépare d'abord en doses unitaires séparées un agent fibrinolytique et un analogue de carbacyclines et on mélange les deux dans un rapport pondéral du fibrinolytique à la carbacycline compris entre 10⁸ et 10².

2. Procédé selon la revendication 1, caractérisé en ce que l'on prépare une dose unitaire de carbacycline contenant de 10ng à 1mg d'iloprost ou une quantité biologiquement équivalente d'une autre carbacycline.

3. Procédé selon la revendication 1, caractérisé en ce que l'on prépare une dose unitaire du fibrinolytique contenant de 5000 à 10⁷ UI de streptokinase ou une quantité biologiquement équivalente d'un autre fibrinolytique.
